# EUROPEAN PATENT APPLICATION

(11) **EP 2 740 371 A1**
(43) Date of publication of application: **11.06.2014**
(21) Application number: 12882886.0
(22) Date of filing: 31.08.2012
(51) Int. Cl.: A24F 47/00

(54) **ELECTRONIC CIGARETTE AND ELECTRONIC CIGARETTE DEVICE**

(71) Applicant: Liu, Qiuming, Shenzhen, Guangdong 518000 (CN)
(72) Inventor: Liu, Qiuming, Shenzhen, Guangdong 518000 (CN)
(74) Representative: Beetz & Partner
(86) International application number: PCT/CN2012/080843
(87) International publication number: WO 2014/032275

(57) **Abstract**

This invention discloses an electronic cigarette and an electronic cigarette device, the electronic cigarette includes an inhaling rod and a power rod, the inhaling rod defines atomizing chambers therein, the power rod includes a power supply therein, the atomizing chambers are mutually parallel to each other, the electronic cigarette further comprises a controller connecting the power supply and the atomizing chambers to control the atomizing chambers to work. The electronic cigarette and the electronic cigarette device of the invention have multiple atomizing chambers parallel to each other, and the controller 300 which connects the power supply 202 and the atomizing chambers to control the atomizing chambers to work, thereby achieving volume and thick smoke for good flavor. On the other hand, the atomizing chambers accommodate different types of cigarette liquid, this makes the electronic cigarette to generate smoke with multiple mixed flavors, thus, the users can change the flavors of smoke as they like.

## Description

### TECHNICAL FIELD

This invention relates to an electronic cigarette and an electronic cigarette device.

### DESCRIPTION OF BACKGROUND

Electronic cigarettes are a kind of simulating cigarettes which heat and atomize materials with flavors through heating wire to generate smoke for users' suction. Current electronic cigarettes usually have only one atomizing chamber, thus available mist is relative thin, and taste is lighter.

In addition, current electronic cigarettes have single taste, which cannot meet users' needs of multi-flavors.

### SUMMARY

An object of the present invention is to provide an electronic cigarette and an electronic cigarette device which can generate heavy and thick smoke with good flavor.

Another object of the present invention is to provide an electronic cigarette and an electronic cigarette device which can generate multiple mixed-favor smoke, so that users can change the flavors of smoke as they like.

To achieve the above objects, the present invention provides an electronic cigarette, comprising an inhaling rod and a power rod, the inhaling rod defining atomizing chambers therein, the power rod comprising a power supply therein, the atomizing chambers being mutually parallel to each other, and the electronic cigarette further comprising a controller which connects the power supply and the atomizing chambers to control the atomizing chambers to work.

Furthermore, the controller comprises: a switch unit which generates a trigger signal in response to an external operation; and an integrated circuit electrically connecting to the switch unit and achieving an electrical connection of the power supply to the atomizing chambers according to the trigger signal.

Furthermore, the switch unit is a micro-mechanical switch or an air flow sensor.

Furthermore, each atomizing chamber is defined in an atomizer, and the atomizers are mutually parallel to each other.

Furthermore, the atomizing chambers accommodate cigarette liquid, and tastes of cigarette liquid in atomizing chambers are different from each other.

Furthermore, the inhaling rod comprises an inhaling sheath, atomizers configured within the inhaling sheath, a mouthpiece disposed at an end of the inhaling sheath, and a first connecting sleeve disposed at another end of the inhaling sheath for engaging with the power rod.

Furthermore, the power rod comprises a power sheath, a power supply disposed in the power sheath, a second connecting sleeve disposed at an end of the power sheath and correspondingly connected to the first connecting sleeve and an end cap disposed at another end of the power sheath.

Furthermore, the second connecting sleeve is fixed with a fixing holder therein, and the switch unit is secured onto the fixing holder.

Furthermore, the integrated circuit is disposed on a circuit board, and the circuit board is fixed on the fixing holder and electrically connected to the switch unit.

Furthermore, the second connecting sleeve in its end which corresponds to the inhaling rod defines connecting tunnels which are consistent with the atomizers in number, and the connecting tunnels are provided with inner threads, and the atomizers are provided with outer threads at an end thereof to correspondingly engage with the inner threads.

Furthermore, the atomizers each comprise a shell, a tubular cigarette liquid reservoir disposed within the shell, a filling layer disposed between the cigarette liquid reservoir and the shell, the shell has one end thereof provided with a lid correspondingly sealing one end of the cigarette liquid reservoir, and the shell has the other end thereof provided with a seat correspondingly sealing the other end of the cigarette liquid reservoir, and outside of the other end of the shell is provided with outer threads.

Furthermore, the connecting tunnel is coaxially provided with a first electrode electrically connected to the circuit board and the corresponding atomizer; and the cigarette liquid reservoir accommodates cigarette liquid therein and is provided with a heating wire, and a second electrode is provided within the seat, the seat is coaxially set a second electrode corresponding to the first electrode, and the second electrode is electrically connected with its corresponding the heating wire.

Furthermore, a sealed chamber in the cigarette liquid reservoir, cigarette liquid accommodated in the sealed chamber, and the heating wire disposed in the sealed chamber together construct the atomizing chamber.

Furthermore, a first insulating sleeve is inserted between the first electrode and the corresponding connecting tunnel, and a second insulating sleeve is inserted between the second electrode and the other end of the shell.

Correspondingly, an electronic cigarette device in accordance with embodiments of the invention is further provided, comprising the above electronic cigarette and an electronic cigarette casing for accommodating and charging the electronic cigarette.

The advantages of the electronic cigarette and the electronic cigarette device of the invention are: by the multiple and parallel atomizing chambers and the controller connecting the power supply with the atomizing chambers to control the work of the atomizing chambers, volume and thick smoke with good flavors is available. In addition, the atomizing chambers each accommodate different types of cigarette liquid, therefore, the electronic cigarette can generate smoke with multiple mixed flavors, and the user can adjust the smoke flavor himself according to his desire.

### DESCRIPTION OF THE DRAWINGS

FIG. 1 is an exploded view of an electronic cigarette in accordance with an embodiment of the present invention.
FIG. 2 is a cross-sectional view of the electronic cigarette of FIG. 1.
FIG. 3 is a cross-sectional view of an electronic cigarette in accordance with another embodiment of the present invention.
FIG. 4 is a cross-sectional view of an atomizer of an electronic cigarette in accordance with an embodiment of the present invention.
FIG. 5 is a circuit block diagram of an electronic cigarette in accordance with an embodiment of the present invention.

### DETAILED DESCRIPTION OF THE EMBODIMENTS

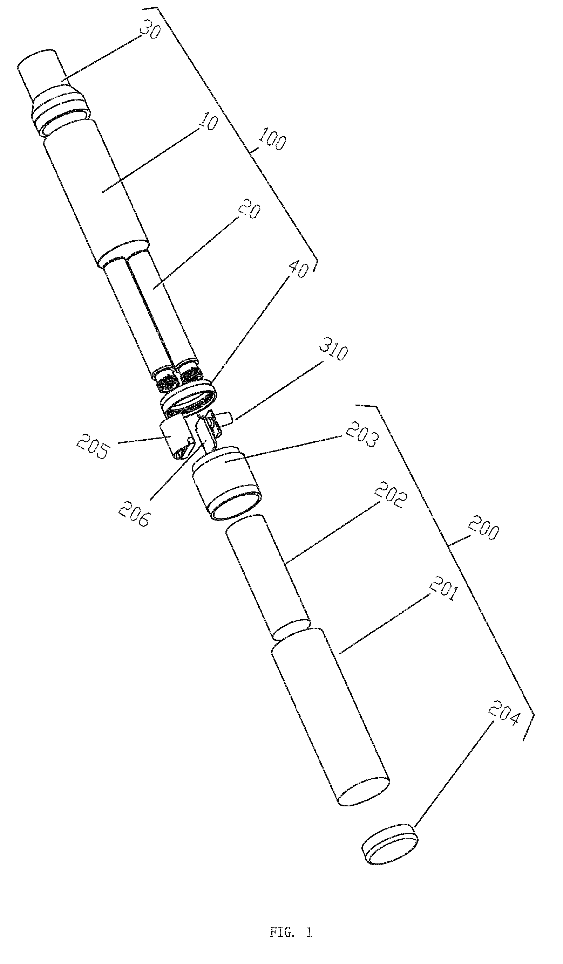

As shown in FIGS. 1-5, an electronic cigarette and an electronic cigarette device are provided in accordance with embodiments of the present invention. The electronic cigarette device comprises an electronic cigarette and an electronic cigarette casing for accommodating and charging the electronic cigarette.

The electronic cigarette provided in the embodiments of the present invention, comprises an inhaling rod 100, a power rod 200 and a controller 300. The inhaling rod 100 comprises an inhaling sheath 10, atomizers 20, a mouthpiece 30 and a first connecting sleeve 40.

The inhaling sheath 10 is in a tubular shape.

The atomizers 20 are configured within the inhaling rod 100, specifically within the inhaling sheath 10, and there are multiple atomizers 20 parallel to each other. Each atomizer 20 is equipped with one atomizing chamber, thus the atomizing chambers are also parallel to each other, and the atomizing chambers accommodate cigarette liquid therein. In one embodiment, all the atomizing chambers have the same types of cigarette liquid; therefore, the controller 300 can control one or more atomizing chambers to work for thick smoke and better taste. In another embodiment, each atomizing chamber has different type of cigarette liquid, for example, cigarette liquid respectively with cigar flavor, Hongtashan^{®} flavor, and Chung Hwa^{®} flavor, etc.. Therefore, the user can enjoy a multiple mixed flavor via multiple atomizing chambers each with different types of cigarette liquid.

The atomizer 20 comprises a shell 21, a cigarette liquid reservoir 22, a filling layer 23, a lid 24 and a seat 25.

The shell 21 is tubular, and the shell 21 is made of steel material.

Cigarette liquid reservoir 22 is tubular, and disposed within the shell 21. In the embodiment, cigarette liquid reservoir 22 is a fiberglass tube, and the cigarette liquid reservoir 22 accommodates cigarette liquid therein and is provided with one or more heating wires 221.

The filling layer 23 is disposed between cigarette liquid reservoir 22 and the shell 21, and the filling layer 23 is embodied as cotton material with buffering and permeability function.

The lid 24 is disposed at an end of the shell 21, and fittingly seals an end of the cigarette liquid reservoir 22.

The seat 25 is disposed within another end of the shell 21, and fittingly seals another end of the cigarette liquid reservoir 22. Said another end of the shell 21 is provided with outer threads.

The mouthpiece 30 is disposed at an end of the inhaling sheath 10, for facilitating users' suction.

The first connecting sleeve 40 is disposed at another end of the inhaling sheath 10, for engaging with the power rod 200.

The power rod 200 comprises a power sheath 201, a power supply 202, a second connecting sleeve 203 and an end cap 204.

The power sheath 201 is tubular, and is made of steel material.

The power supply 202 is disposed in the power sheath 201, and the power supply 202 may be a rechargeable battery or a disposable battery.

The second connecting sleeve 203 is disposed at an end of the power sheath 201, and correspondingly connected to the first connecting sleeve 40. A fixing holder 205 is secured in the second connecting sleeve 203. The fixing holder 205 is fixed with a circuit board 206 thereon, and the circuit board 206 is connected to the power supply 202. The second connecting sleeve 203 in its end which corresponds to the inhaling rod 100 defines connecting tunnels 207 which are consistent with the atomizers 20 in number. Each connecting tunnel 207 is coaxially set with a first electrode 208 therein electrically connected to the circuit board 206 and the corresponding atomizer 20. The connecting tunnel 207 is provided with inner threads, and the atomizer 20 is provided with outer threads at an end thereof to correspondingly engage with the inner threads, therefore, the atomizers 20 are secured to the power rod 200 by means of engagement between the inner threads and the outer threads. Correspondingly, the seat 25 is coaxially provided with a second electrode 251 therein coupled to the first electrode 208. The second electrode 251 is electrically connected with the heating wire 221, so that an electronic connection of the atomizer 20, the circuit board 206 and the power supply 202 is achieved via the first electrode 208 and the second electrode 251. Herein, a sealed chamber of the cigarette liquid reservoir 22, cigarette liquid accommodated in the sealed chamber and the heating wire 221 disposed in the sealed chamber together construct the atomizing chamber. A first insulating sleeve 209 is inserted between the first electrode 208 and the corresponding connecting tunnel 207 for sealing and electronic insulation. A second insulating sleeve 210 is configured between the second electrode 251 and the other end of the shell 21, for sealing and electronic insulation.

The end cap 204 is disposed at the other end of the power sheath 201.

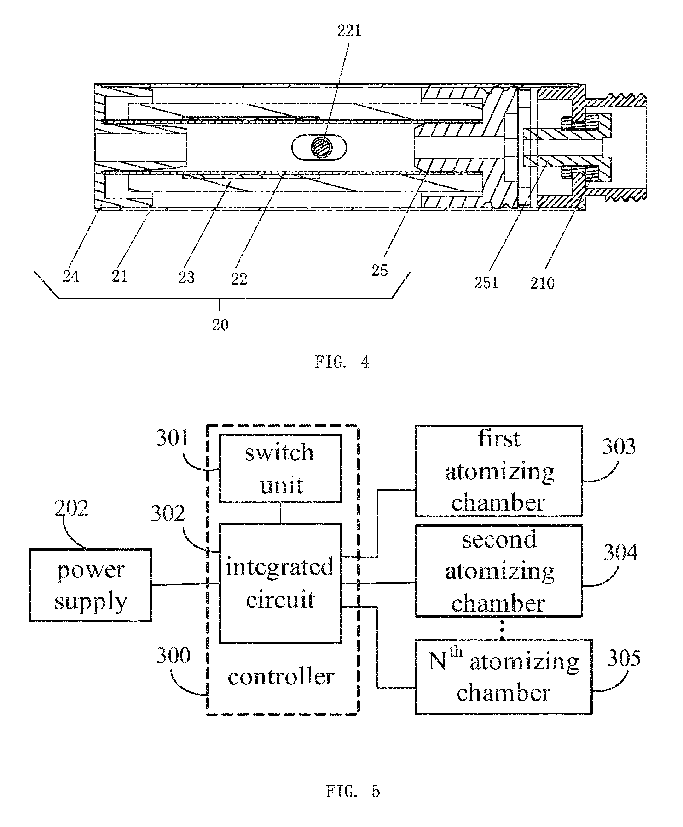

Referring to FIG. 5, the controller 300 connects the power supply 202 and the atomizers 20 () so as to control the atomizing chambers to work. The controller 300 comprises a switch unit 301 and an integrated circuit (IC) 302. In the embodiment, there are N atomizers 20, respectively , a first atomizing chamber 303, a second atomizing chamber 304, ······, and an N^{th} atomizing chamber 305.

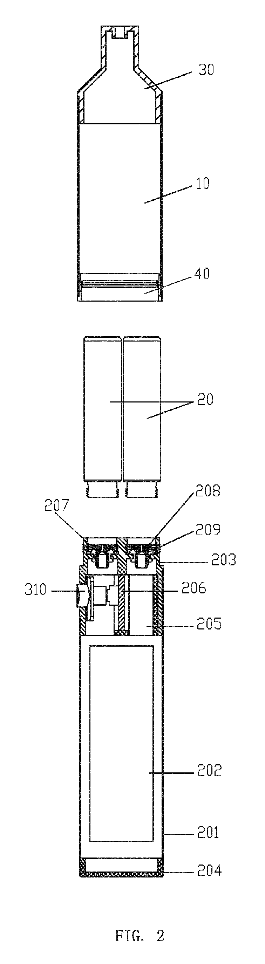

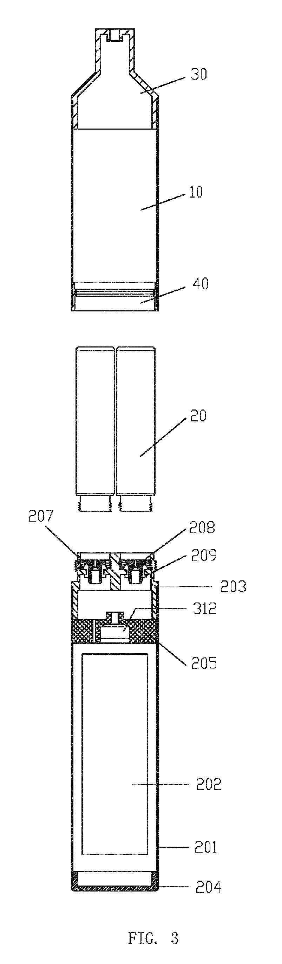

The switch unit 301 is configured to generate a trigger signal in response to an external operation. Specifically, the switch unit 301 is fixed at the fixing holder 205, and the switch unit 301 is, for example, a micro-mechanical switch 310 as shown in FIG. 2, or an air flow sensor 312, i.e., microphone, as shown in FIG. 3. As for the micro-mechanical switch 310, the corresponding trigger signal is generated according to the times or time length of pressing/touching the micro-mechanical switch 310; As for the air flow sensor 312, the corresponding trigger signal is generated according to volume and time length of the air flow.

The IC 302 is electrically connected to the switch unit 301 for achieving an electrical connection of the power supply 202 to the atomizing chambers according to the trigger signal. The IC 302 is disposed on the circuit board 206 which is fixed on the fixing holder 205 and electrically connected to the switch unit 301. The IC 302 is pre-stored with or set up by users with multiple control commands corresponding to the trigger signals, for example, the switch unit 301 receives one pressing of user and generates a trigger signal corresponds to a control command of having the first atomizing chamber 303 and the second atomizing chamber 304 to work simultaneously; a trigger signal generated in response to continuous twice pressing corresponds to a control command of having the first atomizing chamber 303, the second atomizing chamber 304 and a third atomizing chamber (not shown) to work simultaneously. For another example, a trigger signal generated by the switch unit 301 in response to a long pressing of more than 3 seconds corresponds to a control command of having all of the atomizing chambers to work simultaneously, thus, the IC 302 generates the control command corresponding to the trigger signal and the corresponding circuit is conducted according to the control command to have multiple atomizing chambers to work simultaneously and generate volume and thick smoke for good flavor. On the other hand, multiple mixed flavors can be achieved by means of setting control commands, exchanging positions of the atomizers 20, or adding/replacing the atomizers 20 with different types of cigarette liquid.

In summary, the electronic cigarette and the electronic cigarette device of the embodiments of the present invention can achieve volume and thick smoke and better taste by means of multiple parallel atomizing chambers and the controller 300 connecting the power supply 202 with the atomizing chambers to control the atomizing chambers working; on the other hand, the atomizing chambers accommodate different types of cigarette liquid, by which the electronic cigarette can generate smoke with multiple mixed flavors, and the users can change the flavors of smoke as they like.

The above-mentioned is only the embodiments of the present invention. It should be noted, for the persons of ordinary skill in this field, improvements and modifications within the spirit of the present invention can be made, and the improvements and modifications should be seemed to be included in the claimed scope of this invention.

## Claims

1. An electronic cigarette, comprising an inhaling rod and a power rod, the inhaling rod defining atomizing chambers therein, the power rod comprising a power supply therein, **characterized in that**: there are a plurality of atomizing chambers parallel to each other, and the electronic cigarette further comprises a controller which connects the power supply with the atomizing chambers to control the atomizing chambers to work.

2. The electronic cigarette as described in claim 1, **characterized in that**, the controller comprises:
a switch unit which generates a trigger signal in response to an external operation; and
an integrated circuit electrically connecting to the switch unit and achieving an electric connection between the power supply and the atomizing chambers according to the trigger signal.

3. The electronic cigarette as described in claim 2, **characterized in that**: the switch unit is a micro-mechanical switch or an air flow sensor.

4. The electronic cigarette as described in claim 1, **characterized in that**: each atomizing chamber is defined in an atomizer, and the atomizers are mutually parallel to each other.

5. The electronic cigarette as described in claim 1, **characterized in that**: the atomizing chambers accommodate cigarette liquid, and taste of cigarette liquid in each atomizing chamber is different from each other.

6. The electronic cigarette as described in claim 2, **characterized in that**: the inhaling rod comprises an inhaling sheath, the atomizers configured within the inhaling sheath, a mouthpiece disposed at an end of the inhaling sheath, and a first connecting sleeve disposed at the end of the inhaling sheath for engaging with the power rod.

7. The electronic cigarette as described in claim 6, **characterized in that**: the power rod comprises a power sheath, a power supply disposed in the power sheath, a second connecting sleeve disposed at an end of the power sheath and correspondingly connected to the first connecting sleeve, and an end cap disposed at the end of the power sheath.

8. The electronic cigarette as described in claim 7, **characterized in that**: the second connecting sleeve is set with a fixing holder therein, and the switch unit is secured onto the fixing holder.

9. The electronic cigarette as described in claim 8, **characterized in that**: the integrated circuit is disposed on a circuit board, and the circuit board is fixed on the fixing holder and electrically connected to the switch unit.

10. The electronic cigarette as described in claim 6, **characterized in that**: the second connecting sleeve in its end which corresponds to the inhaling rod defines connecting tunnels which are consistent with the atomizers in number, the connecting tunnels are provided with inner threads, and the atomizers are provided with outer threads at an end thereof to correspondingly engage with the inner thread.

11. The electronic cigarette as described in claim 10, **characterized in that**: the atomizers each comprise a shell, a tubular cigarette liquid reservoir disposed within the shell, a filling layer disposed between the cigarette liquid reservoir and the shell, the shell has one end thereof provided with a lid correspondingly sealing one end of the cigarette liquid reservoir, and the shell has the other end inside thereof provided with a seat correspondingly sealing the other end of the cigarette liquid reservoir, and outside of the other end of the shell is provided with outer threads.

12. The electronic cigarette as described in claim 11, **characterized in that**: the connecting tunnel is coaxially provided with a first electrode electrically connected to the circuit board and the corresponding atomizer; the cigarette liquid reservoir accommodates cigarette liquid therein and is provided with a heating wire; and the seat is coaxially provided with a second electrode coupled to the first electrode, and the second electrode is electrically connected with the heating wire.

13. The electronic cigarette as described in claim 12, **characterized in that**: a sealed chamber in the cigarette liquid reservoir, cigarette liquid accommodated in the sealed chamber and the heating wire disposed in the sealed chamber construct the atomizing chamber.

14. The electronic cigarette as described in claim 12, **characterized in that**: a first insulating sleeve is inserted between the first electrode and the corresponding connecting tunnel, and a second insulating sleeve is inserted between the second electrode and the other end of the shell.

15. An electronic cigarette device, **characterized in that**: the electronic cigarette device comprises:
an electronic cigarette as claimed in any one of claims 1-14; and
an electronic cigarette casing for accommodating and charging the electronic cigarette.
